# EUROPEAN PATENT APPLICATION

(11) **EP 1 285 902 A1**
(43) Date of publication of application: **26.02.2003**
(21) Application number: 01932241.1
(22) Date of filing: 24.05.2001
(51) Int. Cl.: C07B 59/00, G01N 33/68, G01N 33/60

(54) **METHOD FOR LABELING WITH TRITIUM**

(30) Priority: 29.05.2000 JP 2000158306
(71) Applicant: SHIONOGI & CO., LTD., Osaka-shi, Osaka 541-0045 (JP)
(72) Inventor: NAGASAKI, Tohru, c/o Shionogi & Co., Ltd, Toyonaka-shi, Osaka 561-0825 (JP)
(74) Representative: Baverstock, Michael George Douglas
(86) International application number: JP0104349
(87) International publication number: WO01092188

(57) **Abstract**

The present invention provides a tritium-labeling method which allows the introduction of tritium at the last stage of preparation and thus the preparation of the tritium-labeled compound having a high specific radioactivity, and said compound prepared by the method.

## Description

### TECHNICAL FIELD

The present invention is related to a method for preparing a tritium-labeled compound characterized by reacting an organic compound with a tritium-labeled hydride reagent in the presence of a solvent and a catalyst, the tritium-labeling method, a tritium-labeled compound obtained according to said production method, a method for establishing pharmacokinetics by using the tritium-labeled compound, a screening method to identify a new useful compound in which the tritium-labeled compound is used as a labeling ligand for a known receptor and a compound obtained by said method, a screening method to identify an unknown protein in which a tritium-labeled compound is used as a labeling agent for a bioactive compound and a protein obtained by said method.

### BACKGROUND ART

A radiolabeled compound is essential to a development of a pharmaceutical agent at various stages as a tracer because it has an excellent sensitivity and is easily detectable. For the purpose of the radiolabeling, a radioisotope such as ¹⁴C (Maximum specific radioactivity about 60 mCi/mmol) and ³H(tritium)(Maximum specific radioactivity about 30 Ci/mmol) are mainly used. Since a tritium-labeled compound has high specific radioactivity and is an excellent tracer, it is especially useful for various experiments in which a labeled compound having high specific radioactivity is required, such as a protein-binding receptor assay.

At present, a tritium-labeled compound is prepared by using a catalytic reduction with a large amount of tritium gas or a conversion with tritium water. The catalytic reduction is usually used rather than the conversion process because in the latter it is difficult to obtain a tritium-labeled compound having high specific radioactivity. However, in the catalytic reduction, when a compound has a functional group such as nitro, a double-bond and benzyl ether the functional group is reduced prior to or simultaneously with tritium-labeling. Accordingly, if interesting tritium-labeled compound has a variety of functional groups, then tritiation must be conducted at a relatively early stage of a synthesis of compound in multi-steps. Such labeling at an early stage requires extensive labor in its practice, because it requires a special synthetic route for synthesis of a labeled compound; a reaction and purification at an ultra-micro scale via difficult several steps; and disposal of a large amount of radwaste from each steps of reaction, frequent radiological monitoring, and complicated removal of by-products. In addition, degradation of the labeled compound may be occurred via several reaction steps, which causes a reduction of the yield. J. Chem. Research (S), 1996, 150-151 discloses a method for obtaining 2,3- or 2,4-dibromotiophene by treating 2,3,5-tribromotiophene with NaBH₄ in the presence of for example palladium catalyst, but a tritium-labeling. In general, in the tritium-labeling, tritium is considered to show a different nature from ¹H due to influence of ultra-micro scale procedure and autolysis by radiation etc.

### SUBJECT TO BE SOLVED

The inventors have studied hard to develop a tritium-labeling method which can be easily conducted and has not such defects as described above, and found that tritium can be introduced at the last stage of synthesis by reacting an organic compound with a tritium-labeled hydride reagent in the presence of a solvent and a catalyst to provide a tritium-labeled compound having high specific radioactivity, whereby the present invention has been accomplished.

The following Schema I illustrates the difference between a tritium-labeling method of the present invention and that in the past by exemplified tritium-labeling of iodo-S-145 methyl ester.

### Schema I

A method of the present invention

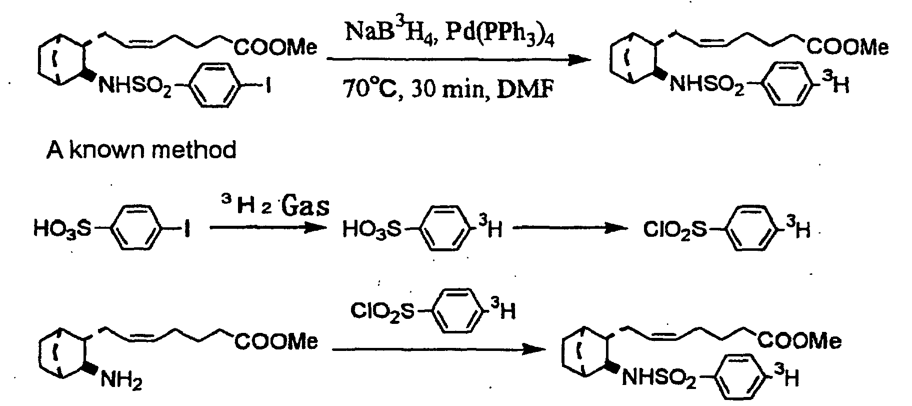

Thus, in the prior art, desired compound is obtained via two difficult ultra-micro scale steps after a building block of S-145 methyl ester is labeled with tritium because said compound has a double-bond which is reduced prior to reduction of a leaving group iodo by catalytic reduction. On the contrary, a method of the present invention allows tritiation at the last stage, whereby a tritium-labeled substance can be easily obtained at a short period of time and then a problem of radwaste has been reduced.

The present invention provides a method for preparing a tritium-labeled compound characterized by reacting an organic compound with a tritium-labeled hydride reagent in the presence of a solvent and a catalyst. The present invention also provides a method for tritiating an organic compound characterized by reacting an organic compound with a tritium-labeled hydride reagent in the presence of a solvent and a catalyst.

The organic compound is preferably, but is not limited to, an organic compound having a leaving group.

An example of a leaving group is, but not limited to, halogen, sulfonyloxy group (optionally substituted alkylsulfonyloxy, optionally substituted haloalkylsulfonyloxy, optionally substituted arylsulfonyloxy), and preferably bromine, iodine, trifluoromethanesulfonyloxy group, methansesulfonyloxy group, p-toluenesulfonyloxy group.

Alkyl means a straight or branched alkyl group having one to 10 carbon atoms, an example of which is for example, methyl, ethyl, n-propyl, isopropyl, n-butyl, sec-butyl, isobutyl, tert-butyl, n-pentyl, n-hexyl, n-heptyl, n-octyl, n-nonanyl, n-decanyl, and preferably methyl, ethyl, and n-propyl.

Alkylsulfonyloxy means sulfonyloxy, wherein the above alkyl is substituted, an example of which is for example methanesulfonyloxy, ethanesulfonyloxy, propanesulfonyloxy,' and preferably methanesulfonyloxy.

Haloalkylsulfonyloxy means a group, wherein a hydrogen atom of the above alkylsulfonyloxy is substituted with one or more halogen, an example of which is for example trifluoromethanesulfonyloxy.

Arylsulfonyloxy means a sulfonyloxy in which phenyl or naphtyl (1-naphtyl, 2-naphtyl) is substituted, an example of which is for example benzenesulfonyloxy, 1-naphthalenesulfonyloxy, 2-naphthalenesulfonyloxy, and preferably benzenesulfonyloxy.

An example of a substituent for an optionally substituted alkylsulfonyloxy, an optionally substituted haloalkylsulfonyloxy, an optionally substituted arylsulfonyloxy is alkyl, aryl etc. An example of a substituted arylsulfonyloxy is for example p-toluenesulfonyloxy group.

An example of a solvent is hexane, toluene, ethyl acetate, tetrahydrofuran, dimethylformamide, dimethylsulfoxide, and preferably dimethylformamide.

An example of a catalyst is a transition metal complex and a metal salt, preferably a transition metal complex, more preferably a palladium catalyst. Examples of a transition metal complex and a metal salt are palladium catalyst, ruthenium catalyst, rhodium catalyst, nickel catalyst, cobalt catalyst, platinum catalyst, iron catalyst, copper catalyst, zinc catalyst, and preferably tetrakis(triphenylphosphine)palladium, palladium acetate, bis(benzonitrile)dichloropalladium(ll), tris(dibenzylidene-acetone)dipalladium(0), dichloro-tris(triphenylphosphine)-ruthenium, chloro-tris(triphenylphosphine)rhodium, nickel(ll) chloride, cobalt(ll) chloride, rhodium(III) chloride. Especially preferred catalyst is tetrakis(triphenylphosphine)palladium.

The amount of catalyst is, but is not limited to, 0.01-100 mol%, particularly 0.1-30 mol%, and more preferably 1-10 mol% of raw material.

An example of tritium-labeled hydride reagent is NaB³H₄, NaB(OMe)₃³H, NaB³H₃CN, NaB(O₂CCH₃)₃³H, LiB³H₄, NaB³H₂S₃, [(CH₃)₂CHCH₂]₂Al³H, LiAl[OC(CH₃)₃]₃³H, preferably, NaB³H₄, NaB(OMe)₃³H, and more preferably NaB³H₄.

The amount of reacting reagent is, but is not limited to, 1-10 equivalents, particularly 1-5 equivalents, and more preferably 2-4 equivalents (all as converted to tritium) to a law material.

This reaction can be conducted under any atmosphere, and especially preferably under an inert gas atmosphere. An example of an inert gas is nitrogen, argon, helium, neon, and preferably nitrogen and argon.

Reaction temperature is, but not limited to, on ice to 150°C, especially room temperature to 120 °C, and more preferably 50-120°C.

The reaction can be conducted under normal, increased or reduced pressure, and in particular preferably under normal pressure. The reaction is to synthesize a tritium-labeled compound, and a residual raw material can be existed. A tritium-labeled compound can be isolated by a procedure used for a usual organic synthesis. For example, silica gel column chromatography , thin layer chromatography, HPLC etc. can be used.

The present invention can be applied to any organic compound, and is useful for tritium-labeling of a compound which is reduced by catalytic reduction. Particularly, it is useful for the labeling of a compound which is reduced by catalytic reduction, and further for a compound of which part other than the intended labeling site (a substituent, a bond etc.) is reduced by catalytic reduction. Thus, in a catalytic reduction of such compound using tritium gas, a part other than the intended labeling site is reduced to be labeled. However, according to the present invention, only the intended part of such compound can be also labeled. In particular, the present invention is useful for a compound contained in a medicament because it has many sites (a substituent, a bond etc.) reduced by a catalytic reduction. Specifically, an example of the compound is the one having nitro group, a triple bond, a double bond, benzyl ether in the molecule and/or one having an aromatic ring to which chlorine or fluorine is bound.

A variety of combination of catalyst and hydride reagent may be applied, and preferably a combination of a palladium catalyst as a catalyst and NaB³H₄ and/or NaB(OMe)₃³H as a tritium-labeled hydride reagent, and more preferably a combination of tetrakistriphenylphosphinepalladium as a catalyst and NaB³H₄ as a tritium-labeled hydride reagent.

For example, according to a tritium-labeling method of the present invention, the labeled compound can be prepared from a compound of the following Formula (A-1)- Formula (F-1).

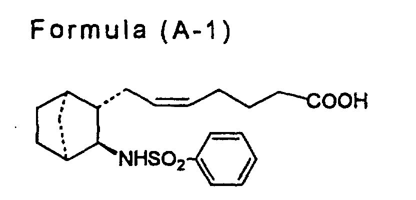

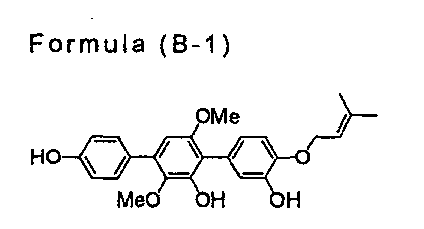

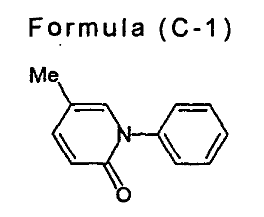

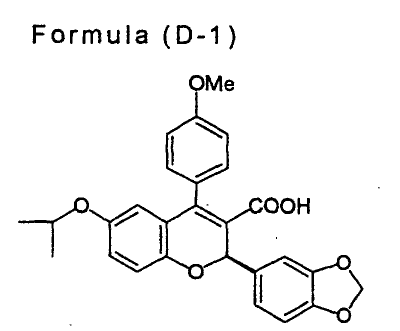

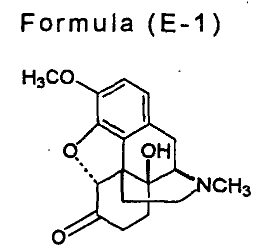

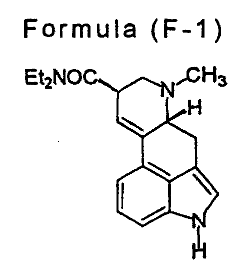

Specifically, the labeled compound of the following Formula (A-2)-Formula (F-2) can be prepared.

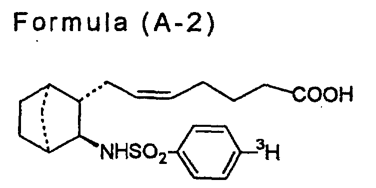

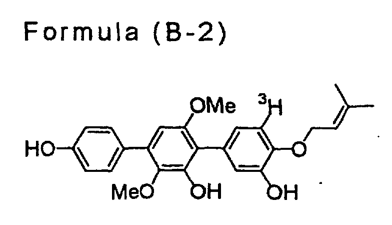

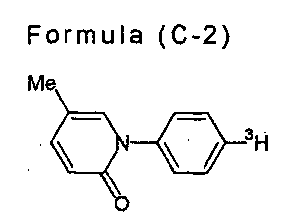

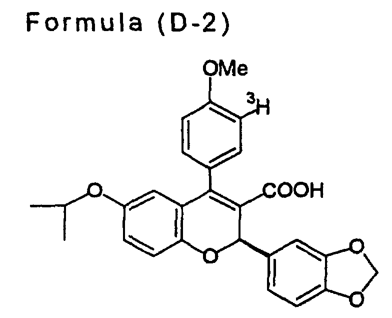

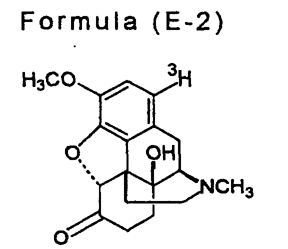

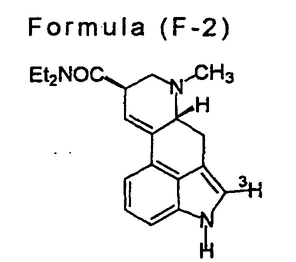

Thus, the present invention also includes a method for preparing a tritium-labeled compound from a compound of Formula (A-1)-Formula (F-1) according to a tritium-labeling method the present invention, and especially a method for preparing a tritium-labeled compound of Formula (A-2)-Formula (F-2).

The present invention also provides a tritium-labeled compound obtained by using a tritium-labeling method as mentioned above. An example of a tritium-labeled compound obtained by the present invention is, but not limited to, for example, a tritium-labeled compound from a compound of Formula (A-1)-Formula (F-1), and especially such as a tritium-labeled compound of Formula (A-2)-Formula (F-2).

The present invention also provides a method for establishing pharmacokinetics using said tritium-labeled compound. For example, a method for establishing a pharmacokinetics using such as a tritium-labeled compound from a compound of Formula (A-1)-Formula (F-1), especially a tritium-labeled compound of Formula (A-2)-Formula (F-2) is within the scope of the present invention.

The present invention also provides a screening method to identify a new useful compound in which said tritium-labeled compound is used as a ligand for labeling a known receptor. For example, a process of screening to identify a new useful compound in which a tritium-labeled compound from a compound of Formula (A-1)-Formula (F-1), especially a tritium-labeled compound of Formula (A-2)-Formula (F-2) is used as a ligand for labeling a known receptor, is also within the scope of the present invention. The present invention also provides an useful compound used for said screening method. An organic compound obtained in said screening method means for example an organic compound with a molecular weight ranging 15-1000, of which a substitutive atom is hydrogen, lithium, boron, carbon, nitrogen, oxygen, fluorine, sodium, magnesium, aluminum, phosphorus, sulfur, chlorine, potassium, calcium, iron, barium, bromine, iodine etc, and especially preferred a compound having a group -C(=O)-, -C(=S)-, -NH-, and/or -OH. A compound having a heterocycle with one to three atoms randomly selected from nitrogen, oxygen, and sulfur is preferred.

The present invention also provides a screening method to identify an unknown protein in which a tritium-labeled compound is used as a labeling substance for a bioactive compound. For example, a screening method to identify an unknown protein in which a tritium-labeled compound from a compound of Formula (A-1)-Formula (F-1), especially a tritium-labeled compound of Formula (A-2)-Formula (F-2) is used as a labeling substance for a bioactive compound. The present invention also provides a protein obtained in said screening method. Particularly, the present invention is useful for research of a receptor which specifically binds to said labeled compound.

An example of a method of studying pharmacokinetics of the present invention is, but is not limited to, autoradiography to quantify a distribution of a substance absorbed in a living body or metabolite thereof in an organ or a tissue with time. For this purpose, saliva, exhalation, urea etc. in addition to an organ or a tissue can be used as a sample (see Hirobe et al. Ed.(1990), Pharmaceutical Research and Development; Vol.18, Drug Metabolism, Hirokawa Publishing Company, Tokyo Japan, "3.2.3 Distribution in Tissues and Autoradiography", pp.103-117).

An example of a screening method of the present invention is, but not limited to, a biochemical method of determining an activity of a sample which influences an activity of an enzyme or ligand-binding activity by using a tissue homogenate, a cell membrane sample, or a partially purified enzyme, or a receptor sample (see Saito et al. Ed.(1990), Pharmaceutical Research and Development; Vol.9, Screening Methods for Drug Evaluation I, Hirokawa Publishing Company, Tokyo Japan, "2.2.2 Biochemical Method", p.20), or histamine receptor binding assay (see Saito et al. Ed.(1990), Pharmaceutical Research and Development; Vol.9, Screening Methods for Drug Evaluation III, Hirokawa Publishing Company, Tokyo Japan, "10.1.3 Histamine Receptor Binding Assay", pp.9-12), and PAF-binding inhibition assay for screening a natural platelet activating factor (PAF) antagonist (see Terada et al. Ed. (1991), The Second Series of Pharmaceutical Research and Development; Vol.5, Separation and Purification of Bioactive Compounds, Hirokawa Publishing Company, Tokyo Japan, "B.PAF-binding Inhibition Assay", pp.308-309) etc. The above references are incorporated herein by reference.

The following examples and examinations further illustrate the present inventions but should not be deemed to limit the scope of the present invention.

### EXAMPLES

### Example 1

A variety of transition metal complexes and metal salts were used as a catalyst for a tritiation of 4-bromobiphenyl as shown in the following Schema I to select a catalyst.

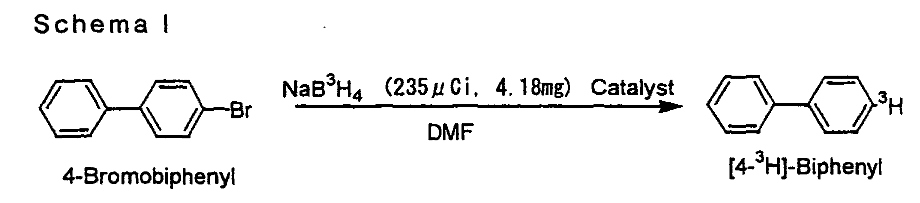

**Table 1:**

| Tritiation of 4-bromobiphenyl using a transition metal complex | | | |
|---|---|---|---|
| Test No. | Catalyst | Total Radio activity (µCi) | Specific Radioactivity Radioactivity (µCi/mmol) |
| 1 | Pd(PPh₃)₄ | 26.4 | 465 |
| 2 | Pd(OAc)₂ | 36.9 | 588 |
| 3 | Pd(PhCN)₂CI₂ | 34.0 | 547 |
| 4 | Pd₂(dba)₃ | 27.0 | 622 |
| 5 | Ru(PPh₃)₃CI | 1.69 | 264 |
| 6 | Rh(PPh₃)₃Cl | 15.6 | 427 |

**Table 2:**

| Tritiation of 4-bromobipheny with a metal salt | | | |
|---|---|---|---|
| Test No. No. | Catalyst | Total Radioactivity (µCi) | Specific Radioactivity (µCi/mmol) |
| 7 | NiCl₂ | 7.08 | 534 |
| 8 | CoOl₂ | 47.6 | 655 |
| 9 | RhCl₃ | 13.3 | 222 |

According to the results shown in Tables 1 and 2, Pd(PPh₃)₄, which provided a stable yield with the highest reproducibility and utility in the study for a variety of compounds, was used for the subsequent tritiation.

### Example 2

A method for synthesizing a tritium-labeled compound is shown in the following Schema II.

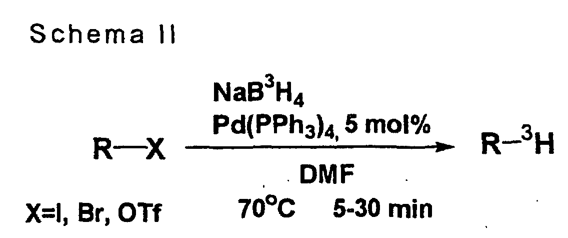

OTf, as used herein, represents trifluoromethanesulfonyloxy group.

### (1) General 3H-Labelling Procedure (Ultra-micro Scale )

A solution of halide compound (18 µmol) and Pd(PPh₃)₄ (2.27 mg, 1.96 µmol) in anhydrous DMF (200 µl) was added onto a solid NaB³H₄ (500 mCi, 0.336 mg, 8.62 µmol, 58 Ci/mmol) in a single portion at room temperature under nitrogen atmosphere. After being allowed to stand at 70°C for 30 min, the mixture was poured into cold water (5 ml) and extracted with ethyl acetate (5 ml x 2). The extracts were washed with water (3 ml x 2), dried over sodium sulfate and concentrated below 30 °C under reduced pressure to about 0.5 ml. From the concentrate, a pure tritium labelled compound was obtained by means of silica gel column chromatography, preparative thin layer chromatography and/or preparative HPLC.

### (2) General Tracer ³H-Labelling Procedure (Micro Scale)

To a stirred solution of halide compound (0.1 mmol) and 5 mol % of catalyst (0.005 mmol) in anhydrous DMF (1.0 ml) was added a solution of NaB³H₄ (235 µCi, 4.18 mg, 0.1 mmol, 2.35 mCi/mmol) at room temperature under nitrogen atmosphere. After being allowed to stand at 70°C for 5-30 min, the mixture was poured into cold water(10 ml) and extracted with ethyl acetate(20 ml). The extract was washed with water (10 ml x 2), dried over sodium sulfate and concentrated below 30 °C under reduced pressure to about 0.5 ml. From the concentrate, a pure tritium labelled compound was obtained by means of column chromatography, preparative thin layer chromatography and/or preparative HPLC.

### Example 3

By using a variety of substrates, tritium-labeling of functional group thereof was conducted.

### (1) Tritium-labeling of an aryl halide type

As shown in the following Schema III, a tritium-labeling test for an aryl halide type was conducted. The results thereof are shown in Table 3. 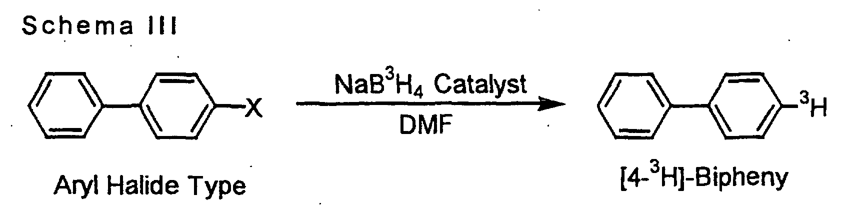 wherein X is Br, I, or OTf.

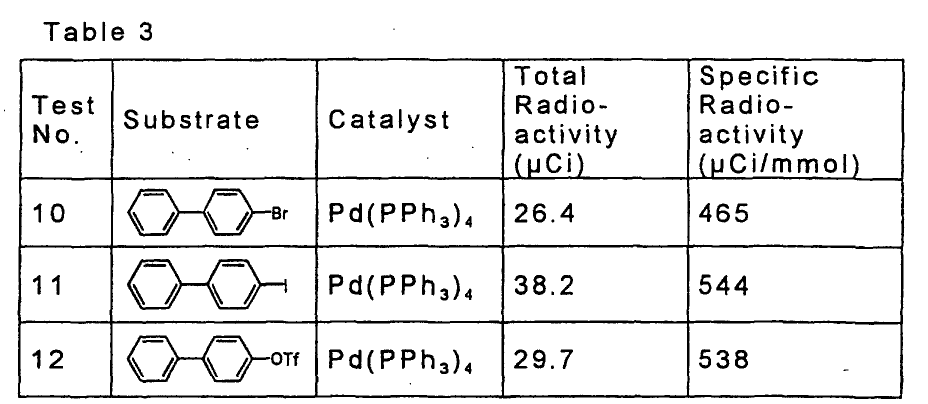

### (2) Tritium-labeling of benzyl halide type

As shown in the following Schema IV, a tritium-labeling test for a benzyl halide type was conducted. The results thereof are shown in Table 4.

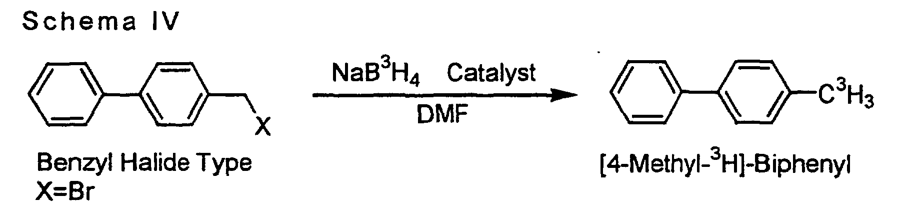

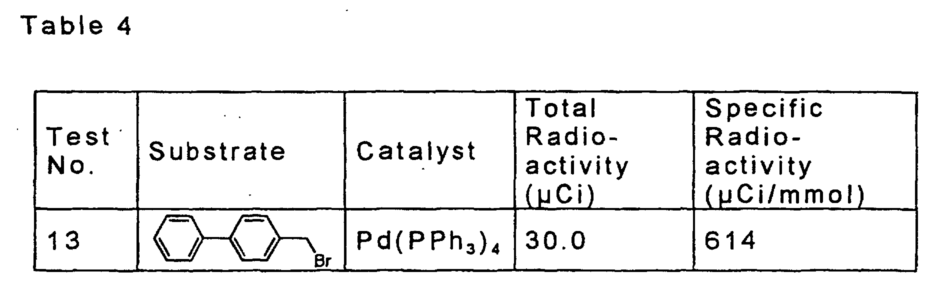

### (3) Tritium-labeling of phenethyl halide type

As shown in the following Schema V, a tritium-labeling test for phenethyl halide type was conducted. The results thereof are shown in Table 5.

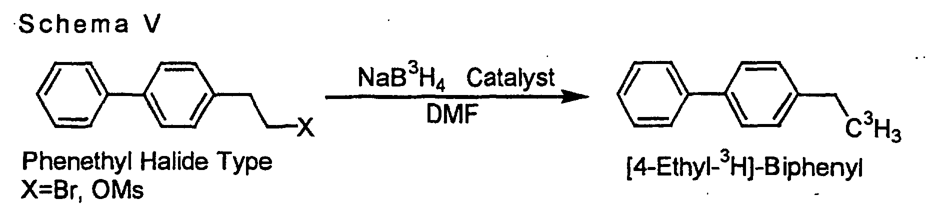

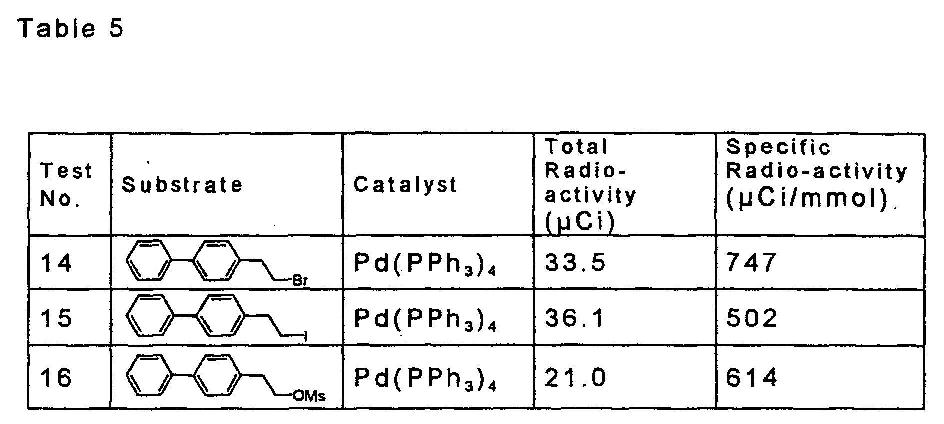

### (4) Tritium-labeling of an alkyl halide type

As shown in the following Schema VI, a tritium-labeling test for an alkyl halide type was conducted. The results thereof are shown in Table 6.

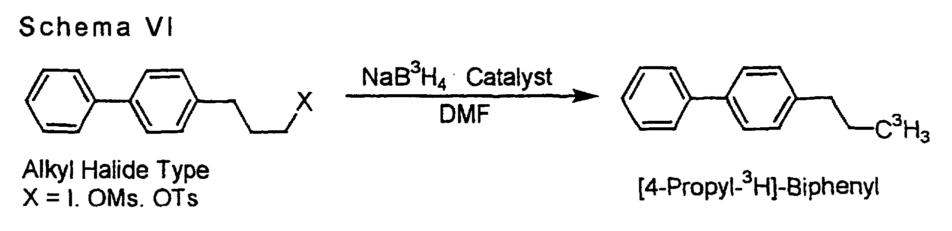

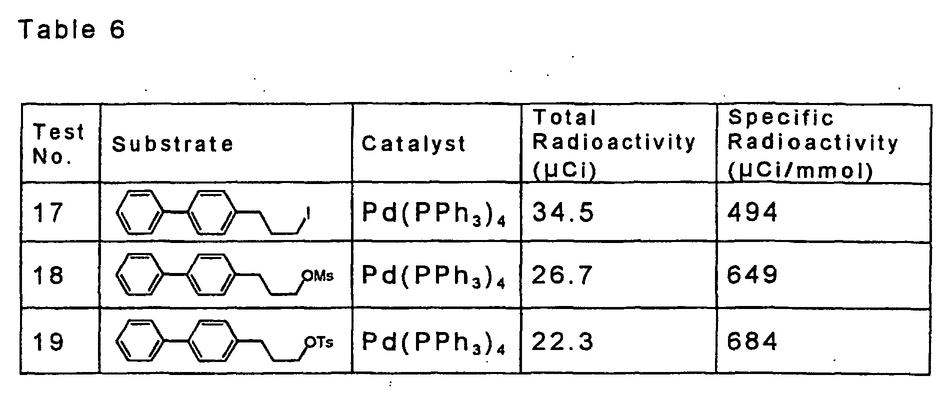

OMs as used herein designates methanesulfonyloxy group and OTs designates p-toluenesulfonyloxy group.

### Example 4

### Preparation of [³H]-S-145 Methyl Ester

A solution of halide, iodo-S-145-methyl ester (13.6 mg, 26.3 µmol) and Pd(PPh₃)₄ (1.52 mg, 1.32 µmol) in anhydrous DMF (250 µl) was added onto a solid NaB³H₄ (500 mCi, 0.326 mg, 8.62 µmol, 58 Ci/mmol) in a single portion at room temperature under nitrogen atmosphere. After being allowed to stand at 70°C for 30 min, the mixture was poured into cold water (5 ml) and extracted with ethyl acetate (7 ml x 2). The extracts were washed with water (5 ml x 2), dried over sodium sulfate and concentrated below 30°C under reduced pressure to give a residue. From the residual oil, a pure tritium labeled compound [[³H]-S-145 methyl ester) was obtained by means of silica gel column chromatography (see the following Schema VII). The results thereof are shown in Table 7.

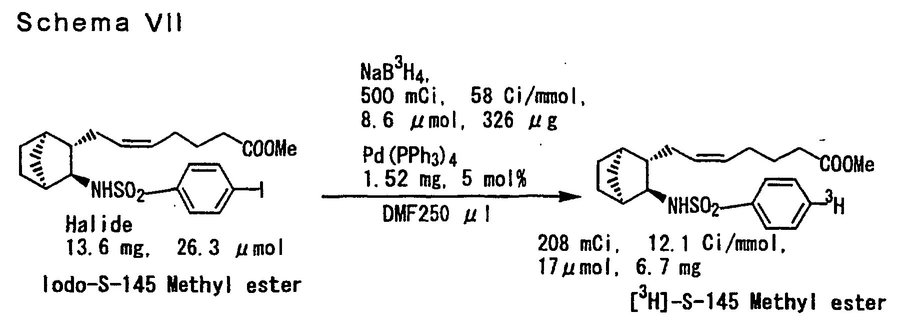

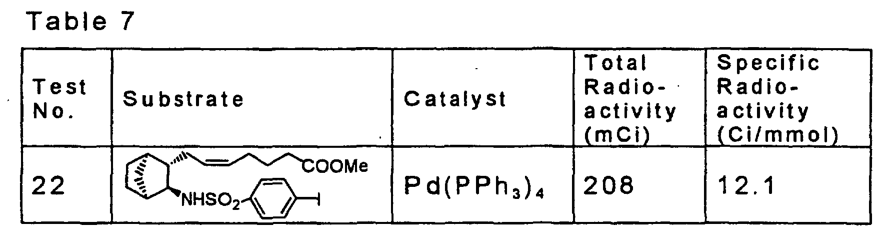

### Example 5

### Synthetic Scheme of [³H]-Terprenin

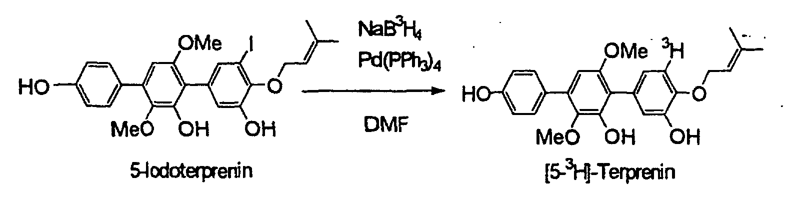

### [³H]-Terprenin

A solution of 5-iodoterprenin (7.3 mg, 13 µmol) in anhydrous DMF (150 µl) was added in a single portion onto a mixture of solid NaB³H₄ (500 mCi, 267 µg, 6.67 µmol, 75 Ci/mmol) and Pd(PPh₃)₄ (1.9 mg, 1.6 µmol) at room temperature under nitrogen atmosphere. After being allowed to stand at 70°C for 30 min, the mixture was poured into cold water (2 ml) and extracted with ethyl acetate (5 ml x 2). The extracts were washed with aqueous sodium chloride solution (3 ml x 2), dried over sodium sulfate and concentrated below 30°C under reduced pressure to give a residue. The residue was dissolved immediately in n-hexane-ethyl acetate (3:1, 0.5 ml) and purified by column chromatography (Merck silica gel No.7734; 300 mg, elution with n-hexane-ethyl acetate (3:1, 1.5 ml/fraction). The fractions containing [³H]-terprenin were combined and evaporated and the residue was purified furthermore by preparative HPLC (Column; Cosmosil 5C18 AR 4.6 mm x 15 cm, Mobile Phase; MeOH:H₂O = 7:3, 1 ml/min, UV; 280 nm, Retention Time; ca. 4.8 min) to give [³H]-Terprenin (6.7 mCi, 260 µg, 0.616 µmol, 10.9 Ci/mmol, Radiochemical Purity; 98.5%. Chemical Purity; above 99%).

### Example 6

### Synthetic Scheme of [³H]-perfenidone

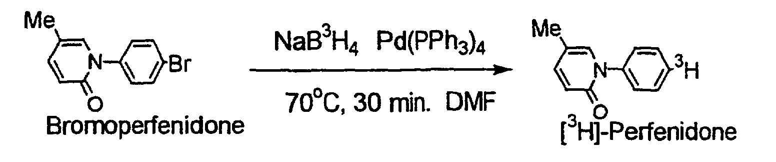

### 5-Methyl-1-[4-3H]-phenyl-2-pyridone ([³H]-perfenidone)

To a solid sodium [³H]-borohydride (100 mCi, 8.47 µmol, 11.8 Ci/mmol) in a small ample was added a solution of 5-methyl-1-(4-bromophenyl)-2-(1H)-pyridone (bromo-perfenidone) (5 mg, 18.9 µmol) and Pd(PPh₃)₄ (3.26 mg, 2.8 µmol) in anhydrous DMF (150 µl) and allowed to stand at 70°C for 1hr. The mixture was poured into ice water (2 ml) and extracted with ethyl acetate (5 ml x 2). The extracts were washed with aqueous sodium chloride (3 ml x 2), dried over sodium sulfate and evaporated in vacuo to give a residue. The residue was purified by preparative TLC (Merck KGF No. 5715, Solvent System: benzene:ethyl acetate = 1:4) to give [³H]-perfenidone (8.33 mCi, 11.2 mCi/mg, 2.08 Ci/mmol, 0.74 mg, radiochemical purity; 99.9%).

### Example 7

### Synthetic Scheme of [³H]-S-1255

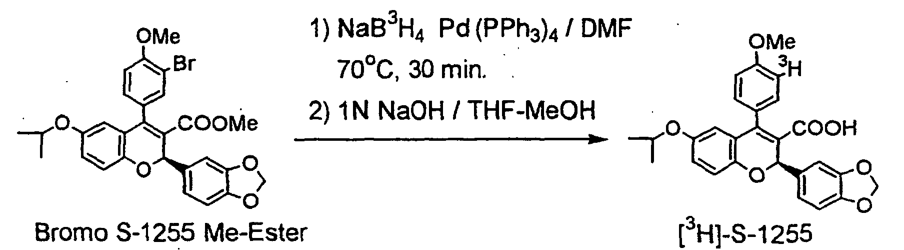

### (R)-(+)-2-(Benzo[1,3]dioxol-5-yl)-6-isopropyloxy-4-(3-[³H]-4-methoxy-phenyl)-4a,8a-dihydro-2H-chromene-3-carboxylic acid ([³H]-S-1255)

To a solid sodium [³H]-borohydride (NaB³H₄) (500 mCi, 253 µg, 6.7 µmol, 75 Ci/mmol) in a small ample were added in a single portion a solution of the bromo-S-1255 methyl ester (7.82 mg, 14.1 µmol) in anhydrous DMF (200 µl and Pd(PPh₃)₄ (1.88 mg, 1.63 µmol) and allowed to stand at 70°C for 1 hr. The reaction mixture was poured into water (6 ml) and extracted with ethyl acetate (7 ml x 2). The extracts were washed with water (5 ml x 2) and aqueous sodium chloride solution (5 ml), dried over sodium sulfate and concentrated in vacuo at 30 °C to give a residue. Purification of the residue by means of flash column chromatography (silica gel Merck No.7734; 1 g, Elution Solvent; ethyl acetate) and preparative TLC (Merck KGF No.5744, Solvent; benzene) gave the intermediate, [³H]-S-1255 methyl ester (25.4 mCi) as a viscous residue. Next, 1 mol/L sodium hydroxide (0.5 ml) was added into a stirred solution of [³H]-S-1255 methyl ester (25.4 mCi) in tetrahydrofuran-methanol (1:1, 3 ml) at 0°C. After being stirred for 1 hr 40 min at 90 °C , the mixture was concentrated to about 0.4 ml, diluted with water (3 ml), acidified with 5% phosphoric acid and extracted immediately with ethyl acetate (7 ml x 2). The extracts were washed with water (2 ml x 2) and aqueous sodium chloride solution (2 ml), dried over sodium sulfate and evaporated in vacuo and almost dried to give a crude [³H]-S-1255 (about 25 mCi) as a residue. The residue was purified by preparative HPLC (Column; Chiral cel OJ-R, 4.6 mm x 15 cm, Mobile Phase; acetonitrile:water: trifluoroacetic acid= 55:45:0.1, 1 ml/min, UV; 286 nm). The fractions containing desired compound were combined and concentrated in vacuo to about 13 ml and extracted with ethyl acetate (7 ml x 2). The extracts were washed with water (3 ml x 2) and aqueous sodium chloride solution (3 ml), dried over sodium sulfate and evaporated in vacuo to give a residue. To prevent deterioration radiochemical purity, the residue was dissolved immediately in methanol (5.0 ml). Labeled compound [³H]-S-1255 (26 mCi, 10.9 Ci/mmol, 23.6 mCi/mg, 5.20 mCi/ml, radiochemical purity: 98.5%) was obtained as methanol solution.

### Example 8

### Synthetic Scheme of [³H]-Oxycodone

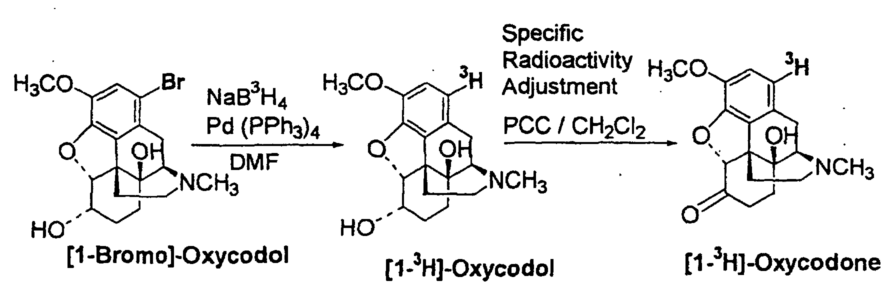

### [1-³H]-4,5α-Epoxy-14-hydroxy-3-methoxy-17-methyl-morphinan-6-one ([1-³H]-oxycodone) via (1-³H]-4,5α-epoxy-6α,14-dihydroxy-3-methoxy-17-methylmorphinane ([1-³H]-6α-oxycodol)

A solution of 1-bromo-6α-oxycodol (2.20 mg, 5.56 µmol) and Pd(PPh₃)₄ (320 µg, 0.278 µmol) in anhydrous DMF (200 µl) was added onto a solid sodium borotritide (500 mCi, 211 µg, 5.55 µmol, 90 Ci/mmol ) in a single portion and allowed to stand at 70 °C for 30 min. Immediately, the carrier unlabelled 6α-oxycodol (6 mg) was added into the reaction mixture to make a working-up and purification easier (Note-1). The mixture was poured into water (3 ml) and extracted with ethyl acetate (5 ml x 2). The extracts were washed with water (2 ml x 2) and aqueous sodium chloride solution (2 ml), dried over Na₂SO₄ and evaporated in vacuo at 35 °C to give a residue as crude [1-3H]-6 α-oxycodol (7 mg). Purification by means of preparative TLC (Merck silica gel KGF₂₅₄ 0.5 mm, Solvent System; CHCI₃: MeOH =7:1 , Extraction Solvent ; CH₂Cl₂:MeOH = 10: 1, 30 ml) gave the intermediate [1-³H]-6 α-Oxycodol (19.8 mCi, 6.3 mg, radiochemical purity; 99.6%). Next, Pyridinium chlorochromate (143 mg, 663 µmol) was added into a stirred solution of [1-³H]-oxycodol (19.8 mCi, 6.3 mg, ca. 20 µmol) and the carrier unlabelled 6 α-oxycodol (35.7 mg, ca.112 µmol) in dichloromethane (10 ml) at room temperature. After being stirred at room temperature for 3 hr, the mixture was poured into a 5% sodium bicarbonate aqueous solution (10 ml) and extracted with ethyl acetate (30 ml x 2). The extracts were washed with 5% sodium bicarbonate (5 ml), water (5 ml x 2) and aqueous sodium chloride solution (2 ml), dried over Na₂SO₄, and evaporated in vacuo at 40 °C to give a crystalline residue as crude [1-³H] oxycodone (ca. 40 mg). Purification by means of preparative TLC (Merck silica gel KGF₂₅₄ 0.5 mm, Solvent System; CHCI₃: MeOH = 7:1, Extraction Solvent; CH₂CI₂,:MeOH = 10:1) gave the title compound [1-³H]-Oxyeodone (32 mg, 15.2 mCi, 150 mCi/mmol, 475 µCi/mg, radiochemical purity 98.7%).
Note-1: If the carrier was not added in the working-up process, [1-³H]-6α-Oxycodol with greater than 10 Ci/mmol of specific radioactivity would be obtained.

### Example 9

### Synthetic Scheme of [³H]-Lysergide ([³H]-LSD)

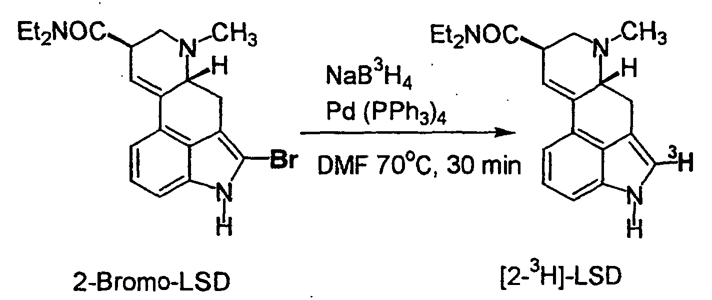

### [³H]-Lysergide ([³H]-LSD)

To a solid sodium [³H]-borohydride (500 mCi, 222 µg, 5.56 µmol, 90 Ci/mmol) in a small ample was added a solution of 2-bromo-LSD (2.24 mg, 5.57 µmol) and Pd(PPh₃)₄ (320 µg, 0.28 µmol) in anhydrous DMF (100 µl) in a single portion and allowed to stand at 70°C for 30 min. After cooling, the mixture was diluted with CH₂Cl₂-DMF (4: 1, 1 ml) and passed through a silica gel column (Merck silica gel KGF No. 9385; 70 mg) and eluted with CH₂Cl₂-DMF (4:1, 1.5 ml) and THF (1.0 ml). The eluates were combined and evaporated in vacuo to give a residue as crude [³H]-LSD (26.7 mCi, Radiochemical Purity ca. 90%). The residue was purified by preparative TLC (Merck TLC plate KGF₂₅₄, Solvent System; acetone: chloroform:methanol = 15:4:1, Extraction Solvent; THF-MeOH = 5:1, 10 ml and Merck TLC plate RP-18F₂₅₄-S, Solvent System: MeCN: H₂O = 85:15, Extraction Solvent: THF:MeOH = 5:1) to give [3H]-LSD (5 mCi, ca. 15.5 Ci/mmol, Radiochemical purity; 96.7%).

By using a tritium-labeled compound which was obtained using a tritium-labeling method of the present invention, the following TXA₂ receptor binding examination was conducted.

### Examination 1

### Preparation of a fraction of human platelet membrane

Blood which was obtained using a plastic syringe contained 3.8% sodium citrate from the vein of normal human (adult male and female) was put into a plastic tube, and after lightly mixing with tumbling, centrifuged at 1800 rpm, for 10 min. at a room temperature to obtain platelet rich plasma (PRP) in supernatant. The PRP was further centrifuged at 2,300 rpm, for 22 min. at a room temperature to obtain platelets. The obtained platelets was homogenized using homogenizer (Ultra-Turrax), and then, centrifuged at 20,000 rpm, for 10 min. at 4°C to obtain a fraction of platelet membrane. The membrane fraction was subjected to a quantitative assay for a protein to be adjusted to 2 mg/ml, and kept at -80°C in a freezer until a binding assay is conducted.

### Examination 2

### TXA₂ receptor binding assay

### (1 )Preparation of human platelet membrane fraction.

According to a procedure as described in Examination. 1, human platelet membrane fraction was prepared.

### (2)TXA₂ receptor binding assay

To 0.2 ml of a binding reaction mixture (50 mM Tris/HCI, pH 7.4, 10 mM MgCI₂), human platelet membrane fraction (0.05mg) and 26.4 Ci/mmol of 2nM [³H](+)-(5Z)-7-[3-endo-[(phenylsulfonyl)amino]bicyclo[2.2.1]hepto-2-exo-yl] heptenic acid sodium salt (Japanese Patent Publication No. H5-79060, referred hereinafter to (+)-S-145 sodium salt) were added, and reacted for 90 min at room temperature. After reaction, the reaction was filtered by using grass fiber filter, washed several times with cold saline, and then, radioactivity remaining on the filter was assayed. The amount of specific binding was calculated by subtracting the amount of non-specific binding (radioactivity obtained in the same manner in the presence of 10 µM (+)-S-145 sodium salt) from the total amount of binding. An activity to inhibit a binding of a test compound was obtained by regarding the amount bound in the absence of a compound as 100%, and obtaining the amount (%) bound in the presence of a test compound to prepare a substitution curve, and then, calculating 50% inhibitory concentration (IC₅₀ value). The results are provided below.

| Compound No. | TXA₂ receptor-binding inhibition activity IC₅₀(µM) |
|---|---|
| 1 | 0.15 |
| 2 | 3.8 |

The test compounds used in this binding assay (Compound Nos.1-2) were prepared according to the following Schema VIII.

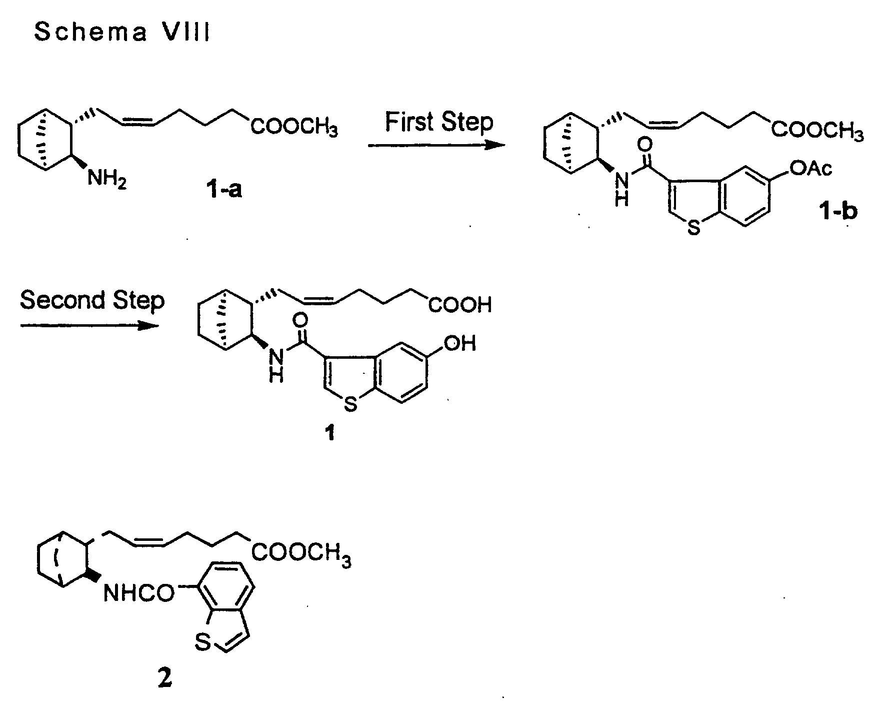

## Claims

1. A method for preparing a tritium-labeled compound **characterized by** reacting an organic compound with a tritium-labeled hydride reagent in the presence of a solvent and a catalyst.

2. A method as claimed in Claim 1 **characterized by** reacting an organic compound which has a leaving group with a tritium-labeled hydride reagent in the presence of a solvent and a catalyst to substitute the leaving group with tritium.

3. A method as claimed in Claim 1 or 2 wherein the organic compound is a compound reduced by a catalytic reduction.

4. A method as claimed in Claim 3 wherein the compound reduced by a catalytic reduction is a compound having a nitro group, triple bond, double bond, and/or benzyl ether in its molecule.

5. A method as claimed in Claim 1 or 2 wherein the catalyst is a palladium catalyst.

6. A method as claimed in Claim 5 wherein the palladium catalyst is tetrakis(triphenylphosphine)palladium.

7. A method as claimed in any one of Claims 1, 2, 4 or 6 wherein the tritium-labeled hydride reagent is NaB³H₄ and/or NaB(OMe)₃³H.

8. A method as claimed in Claim 7 wherein the tritium-labeled hydride reagent is NaB³H₄.

9. A method as claimed in any one of Claims 2, 4, 6 or 8 wherein the leaving group is halogen or sulfonyloxy group.

10. A method as claimed in any one of Claims 1, 2, 4, 6 or 8 wherein the process is conducted under an inert gas atmosphere.

11. A method as claimed in Claim 10 wherein the inert gas is nitrogen or argon.

12. A method as claimed in any one of Claims 1, 2, 4, 6, 8 or 11 wherein the solvent is dimethylformamide.

13. A tritium-labeled compound which is obtained according to a method as claimed in any one of Claims 1, 2, 4, 6, 8 or 11.

14. A method for establishing pharmacokinetics in which a tritium-labeled compound obtained according to a method as claimed in any one of Claims 1, 2, 4, 6, 8 or 11 is used.

15. A screening method for identifying a new useful compound in which a tritium-labeled compound obtained according to a method as claimed in any one of Claims 1, 2, 4, 6, 8 or 11 is used as a labeling ligand for a known receptor.

16. A screening method of identifying an unknown protein in which a tritium-labeled compound obtained according to a method as claimed in any one of Claims 1, 2, 4, 6, 8 or 11 is used as a labeling substance for a bioactive compound.

17. A compound which is obtained according to a screening method as claimed in Claim 15.

18. A protein which is obtained according to a screening method as claimed in Claim 16.

19. A method for tritium-labeling an organic compound **characterized by** reacting an organic compound with a tritium-labeled hydride reagent in the presence of a solvent and a catalyst.
